# EUROPÄISCHE PATENTSCHRIFT

(11) **EP 1 159 237 B1**
(45) Veröffentlichungstag und Bekanntmachung des Hinweises auf die Patenterteilung: **10.09.2003**
(21) Anmeldenummer: 00909324.6
(22) Anmeldetag: 06.03.2000
(51) Int. Cl.: C07C 2/10, C07C 11/02, C07C 29/16, C07C 31/125, C07C 41/03, C07C 43/13, C07F 9/11, C07F 9/09

(54) **VERFAHREN ZUR HERSTELLUNG VON TENSIDALKOHOLEN UND TENSIDALKOHOLETHERN, DIE HERGESTELLTEN PRODUKTE UND IHRE VERWENDUNG**
METHOD OF PREPARING SURFACE-ACTIVE ALCOHOLS AND SURFACE-ACTIVE ALCOHOLIC ETHERS, PRODUCTS OBTAINED BY SAID METHOD AND THEIR USE
PROCEDE DE PREPARATION D'ALCOOLS TENSIOACTIFS ET D'ETHERS D'ALCOOLS TENSIOACTIFS, PRODUITS OBTENUS ET LEUR UTILISATION

(30) Priorität: 09.03.1999 DE 19910370
(43) Veröffentlichungstag der Anmeldung: 05.12.2001
(73) Patentinhaber: BASF AKTIENGESELLSCHAFT, 67056 Ludwigshafen (DE)
(72) Erfinder: MAAS, Heiko, D-67105 Schifferstadt (DE); RÖPER, Michael, D-67157 Wachenheim (DE); WALTER, Marc, D-67227 Frankenthal (DE); SCHULZ, Ralf, D-67346 Speyer (DE); TROPSCH, Jürgen, D-67354 Römerberg (DE); JÄGER, Hans-Ulrich, D-67434 Neustadt (DE)
(74) Vertreter: Isenbruck, Günter, Dr.
(86) Internationale Anmeldenummer: EP0001935
(87) Internationale Veröffentlichungsnummer: WO00053547

(56) Entgegenhaltungen:
- DE-A- 4 339 713
- DE-A- 19 604 466
- GB-A- 1 471 481
- US-A- 5 780 694

## Beschreibung

Die vorliegende Erfindung betrifft ein Verfahren zur Herstellung von Tensidalkoholen und Tensidalkoholethern, die sich u.a. sehr gut als Tenside bzw. zur Herstellung von Tensiden eignen. Dabei werden, ausgehend von Olefingemischen Gemische mit einem überwiegenden Anteil verzweigter Dodecene hergestellt, die anschließend zu Tensidalkoholen derivatisiert und danach gegebenenfalls alkoxyliert werden.

Die Erfindung betrifft ferner die Verwendung der Tensidalkohole und Tensidalkoholether zur Herstellung von Tensiden durch Glycosidierung oder Polyglycosidierung, Sulfatierung oder Phosphatierung.

Fettalkohole mit Kettenlängen von C₈ bis C₁₈ werden zur Herstellung von nichtionischen Tensiden eingesetzt. Diese werden mit Alkylenoxiden zu den entsprechenden Fettalkoholethoxilaten umgesetzt. (Kap. 2.3 in: Kosswig/Stache, "Die Tenside", Carl Hanser Verlag, München Wien (1993)). Dabei beeinflußt die Kettenlänge des Fettalkohols unterschiedliche Tensideigenschaften wie z.B. Netzvermögen, Schaumbildung, Fettlösevermögen, Reinigungskraft.

Fettalkohole mit Kettenlängen von C₈ bis C₁₈ können auch zur Herstellung von anionischen Tensiden, wie Alkylphosphaten und Alkyletherphosphaten genutzt werden. Anstelle von Phosphaten können auch die entsprechenden Sulfate hergestellt werden. (Kap. 2.2 in: Kosswig/Stache, "Die Tenside", Carl Hanser Verlag, München Wien (1993)).

Solche Fettalkohole sind aus nativen Quellen zugänglich, z.B. aus Fetten und Ölen, oder aber auf synthetischem Weg durch Aufbau aus Bausteinen mit einer geringeren Zahl an Kohlenstoffatomen. Eine Variante dabei ist die Dimerisierung eines Olefins zu einem Produkt mit doppelter Anzahl von Kohlenstoffatomen und seine Funktionalisierung zu einem Alkohol.

Lineare Olefine geeigneter Kettenlänge sind zur Zeit hauptsächlich nach zwei Verfahren zugänglich:

Bei der Fischer-Tropsch-Synthese fallen neben Paraffinen Olefinisomeren-Gemische als Koppelprodukte an.

Als weitere Quelle für die Gewinnung geeigneter Olefine in technischen Umfang hat sich die Ethylen-Oligomerisierung etabliert. Hierbei kommen sowohl Aluminiumalkyle als Katalysatoren zum Einsatz als auch homogene Nickel-Katalysatoren, wie im Falle des bekannten SHOP-Prozesses der Firma Shell (Weissermel/Arpe, Industrielle organische Chemie).

Olefinfraktionen geeigneter Kettenlänge werden zu Tensidalkoholen weiterverarbeitet. Die Verwendung von Ethylen hat den Nachteil hoher Einsatzstoff-Kosten für den Monomerbaustein. Verfahren zur Herstellung von Tensiden, die auf Ethylen als Ausgangsmaterial basieren, sind daher wirtschaftlich kritisch.

Zur Dimerisierung von Olefinen ist eine Reihe von Verfahren bekannt. So kann die Reaktion an einem heterogenen Kobaltoxid/Kohle-Katalysator durchgeführt werden (FR-A-1 403 273), in Gegenwart von Säuren wie Schwefel- oder Phosphorsäure (FR 964 922), Aluminiumalkyl-katalysiert(WO 97/16398), oder mit einem homogen gelösten Nickelkomplex-Katalysator (US-A-4 069 273). Nach den Angaben der US-A-4 069 273 erhält man bei der Verwendung dieser Nickelkomplex-Katalysatoren - wobei als Komplexbildner 1,5-Cyclooctadien oder 1,1,1,5,5,5 Hexafluorpentan-2,4-dion eingesetzt werden - hochgradig lineare Olefine mit hohem Anteil an Dimerisierungsprodukten. In der FR-A-1 274 529 wird die durch Lewis-Säuren katalysierte Dimerisierung von Methylpentenen beschrieben, wobei als Lewis-Säure Bortrifluorid eingesetzt wird. Dieses Verfahren hat den Nachteil, daß es schwierig ist, den Katalysator aus dem Reaktionsprodukt abzutrennen. Dadurch werden nicht nur mit Katalysatorresten verunreinigte Produkte erhalten, sondern es tritt auch ein beachtlicher Katalysatorverlust ein.

Die DE-A43 3 9 713(D1) betrifft ein Verfahten zur Oligomerisierung von unverzweigten C₂ bis C₆-Olefinen und Katalysatoren, die so optimiert worden sind, daß sie bei diesem Verfahren besonders hohe Anteile der dort erwünschten linearen Reakuonsprodukte liefern. In den Ausführungsbeispielen 3 bis 5 werden Butan/Buten-Gemische oligomerisiert, wobei Reaktionsgemische mit 62 bis 78 Gew.-% Octen-Anteilen erhalten werden.

Die Funktionalisierung der Olefine zu Alkoholen unter Erweiterung des Kohlenstoffgerüstes um ein C-Atom erfolgt zweckmäßigerweise über die Hydroformylierungsreaktion, die ein Gemisch von Aldehyden und Alkoholen liefert, welches nachfolgend zu Alkoholen hydriert werden kann. Jährlich werden weltweit etwa 7 mio Tonnen Produkte mit Hilfe der Hydroformylierung von Olefinen hergestellt. Eine Übersicht über Katalysatoren und Reaktionsbedingungen des Hydroformylierungsverfahrens geben zum Beispiel Beller et al. in Journal of Molecular Catalysis, A104 (1995), 17-85 oder auch Ullmanns Encyclopedia of Industrial Chemistry, Bd.A5 (1986), Seite 217 ff., Seite 333, sowie die diesbezüglichen Literaturverweise.

Die GB-A-1 471 481 (D2) betrifft ein Verfahren zur Hydroformylierung von Olefinen unter Verwendung eines Kobalt enthaltenden Katalysators. Die dort eingesetzten Olefine sind linear und liefern demgemäß nur wenig verzweigte Oxoalkohole und -aldehyde.

Die DE-A-196 04 466 betrifft wäßrige Zubereitungen enthaltend ein Alkylpolyglycosid und ein Polyethylenglycolderivat der dort angegebenen Formel I Der in dem Polyglycosid enthaltene Alkylrest R² (Seite 2, Zeile 55) soll 8 bis 18, vorzugsweise 10 bis 16 C-Atome enthalten, über seinen Verzweigungsgrad finden sich keine direkten Angaben. Aus der auf Seite 3, Zeile 11, gemachten Angabe, daß der Alkylrest sich insbesondere von Fettalkoholen ableiten soll, die durch Hydrierung nativer Fettsäuren erhalten werden, ist zu schließen, daß es sich um weitgehend lineare Alkylreste handeln muß.

Aus der WO 98/23566 ist es bekannt, daß Sulfate, Alkoxylate, Alkoxysulfate und Karboxylate einer Mischung verzweigter Alkanole (Oxp-Alkohole) gute Oberflächenaktivität in kaltem Wasser zeigen und gute Bio-Abbaubarkeit aufweisen. Die Alkanole der eingesetzten Mischung haben eine Kettenlänge von über 8 Kohlenstoffatomen, sie weisen im Durchschnitt 0,7 bis 3 Verzweigungen auf. Die Alkanolmischung kann, beispielsweise durch Hydroformylierung, hergestellt werden aus Mischungen verzweigter Olefine, die ihrerseits entweder durch Skelettisomerisierung oder durch Dimerisierung interner, linearer Olefine erhalten werden können.

Als Vorteil des Verfahrens wird genannt, daß zur Herstellung des Dimerisierungs-Feeds kein C₃- oder C₄-Olefin-Strom verwendet wird. Daraus forlgt, daß nach dem derzeitigen Stand der Technik die dort der Dimerisierung unterworfenen Olefine aus Ethylen hergestellt worden sein (z.B. SHOP-Verfahren). Da Ethylen für die Tensidherstellung ein relativ teures Ausgangsmaterial ist, sind Ethylen-basierende Verfahren gegenüber Verfahren, die von C₃- und/oder C₄-Olefin-Strömen ausgehen, wirtschaftlich benachteiligt.

Die Struktur der Komponenten der Oxo-Alkanolmischung richtet sich nach der Art des Olefingemisches, das der Hydroformylierung unterworfen wurde. Olefingemische, die durch Skelettisomerisierung aus Alphaolefingemischen erhalten wurden, führen zu Alkanolen, die überwiegend an den Enden der Hauptkette, d.h. in den Positionen 2 und 3, gerechnet vom jeweiligen Kettenende, verzweigt sind.

Die oberflächenaktiven Endprodukte werden aus den Alkanolmischungen entweder durch Oxydieren der -CH₂OH-Gruppe zur Carboxylgruppe, oder durch Sulfatieren der Alkanole oder ihrer Alkoxylate gewonnen.

Ähnliche Verfahren zur Herstellung von Tensiden werden in der PCT-Patentanmeldung WO 97/38957 und in der EP-A-787 704 beschrieben. Auch bei den dort beschriebenen Verfahren wird ein Alphaolefin zu einem Gemisch vorwiegend vinylidenverzweigter Olefindimerer dimerisiert:

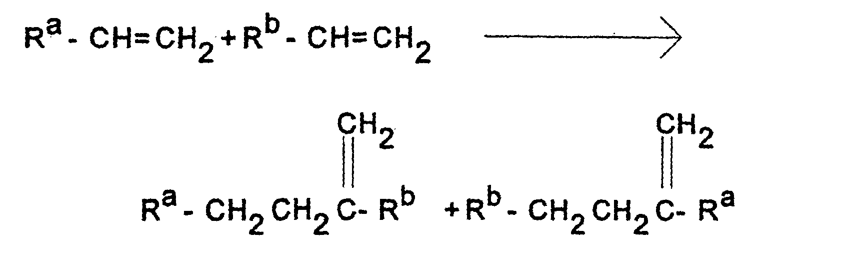

Anschließend werden die Vinylidenverbindungen doppelbindungsisomerisiert, sodaß die Doppelbindung vom Kettenende weiter in die Mitte wandert und anschließend der Hydroformylierung zu einem Oxoalkohol-Gemisch unterworfen. Dieses wird dann weiter, z.B. durch Sulfatierung zu Tensiden umgesetzt. Ein gravierender Nachteil dieses Verfahrens besteht darin, daß es von Alphaolefinen ausgeht. Alphaolefine werden z.B. durch Übergangsmetall-katalysierte Oligomerisierung von Ethylen, Ziegler-Aufbaureaktiön, Wachscracken oder Fischer-Tropsch-Verfahren gewonnen und stellen daher relativ teure Ausgangsmaterialien für die Tensidherstellung dar. Ein weiterer erheblicher Nachteil dieses bekannten Tensid-Herstellungsverfahrens besteht darin, daß bei ihm zwischen der Dimensierung der Alphaolefine und der Hydroformylierung des Dimerisierungsprodukts eine Skelettisomerisierung eingeschaltet werden muß, wenn man zu überwiegend verzweigten Produkten kommen will. Aufgrund der Verwendung eines für die Tensidherstellung relativ teuren Ausgangsmaterials und der Notwendigkeit, einen zusätzlichen Verfahrensschritt, die Isomerisierung, einzuschalten ist dieses bekannte Verfahren wirtschaftlich erheblich benachteiligt.

In der US-A-5,780,694 wird die Herstellung und Verwendung von Alkoholen mit Verzweigungsgraden zwischen 0,9 und 2 beschrieben. Die Alkohole werden durch homogenkatalysierte Dimerisierung von internen Olefinen und nachfolgende Hydroformylierung hergestellt, wobei der n-Anteil in dem zu dimerisierenden Olefin mehr als 85 Gew.-% beträgt. Als besonderer Vorteil dieser Alkohole wird das Kaltwaschverhalten ihrer Sulfate genannt. Über die Eigenschaften der entsprechenden Ethoxylate und deren Sulfate werden in dieser Druckschrift keine Angaben gemacht. Als weiterer Vorteil dieses Verfahrens wird genannt, daß zur Herstellung der Alkohole keine Propen oder Buten enthaltenden Olefingemische verwendet werden, sondern Gemische, die wenigstens 85 Gew.-% C₆ bis C₁₀ Olefine enthalten.

Aus den geschilderten Nachteilen des Standes der Technik, insbesondere auch aus dem Bedürfnis, die Herstellungskosten herabzusetzen, ergibt sich die Aufgabe, ein Verfahren zur Herstellung von anwendungstechnisch vorteilhaften Tensidalkoholen bereitzustellen, bei dem der Einsatz teurer Rohstoffe, insbesondere des teuren Ethylens, vermieden werden kann.

Es wurde nun überraschenderweise gefunden, daß man zu verzweigten Olefinen und Alkoholen (Oxoalkoholen) gelangt, die sich zu sehr gut wirksamen Tensiden weiterverarbeiten lassen - im Folgenden als "Tensidalkohole" bezeichnet-, und die besonders vorteilhafte Eigenschaften im Hinblick auf Ökotoxizität und Bioabbaubarkeit aufweisen, wenn man nach dem im Folgenden beschriebenen erfindungsgemäßen Verfahren arbeitet.

Ein Gegenstand der vorliegenden Erfindung ist somit ein Verfahren zur Herstellung von Tensidalkoholen und entsprechenden Tensidalkoholethern durch
a) Dimerisierung von Olefingemischen,
b) Derivatisierung zu primären Alkoholen, und
c) gegebenenfalls anschließende Alkoxylierung,
das dadurch gekennzeichnet ist, daß man für die Dimerisierung einen Nickel enthaltenden Katalysator und ein Olefingemisch einsetzt, umfassend im wesentlichen C₆ bis C₁₂ - Olefine, das mindestens 55 Gew.-% Hexenisomere enthält, wobei der Hexenisomerenanteil weniger als 30 Gew.-% lineare Isomere aufweist.

Vorzugsweise wird der Verfahrensschritt a), die Dimerisierung, heterogenkatalysiert durchgeführt. Weiterhin ist es bevorzugt, ein Olefingemisch einzusetzen, das mindestens 65 Gew.-% Hexenisomere enthält.

Besonders bevorzugt ist der Einsatz von sogenanntem Dimerpropen als Olefingemisch beim Schritt a) des erfindungsgemäßen Verfahrens. Unter "Dimerpropen" versteht man ein Hexenisomeren-Gemisch, das in Raffinerieprozessen bei der PropenOligomerisierung z.B. nach dem ®DIMERSOL-Verfahren anfällt (Vergl. Cornils/Herrmann, Applied Homogeneous Catalysis, Verlag Chemie (1996)).

Bei der Dimerisierung der Hexenisomeren-Gemische (Schritt a) des erfindungsgemäßen Verfahrens) erhält man Dimerisierungsprodukte, die im Hinblick auf die weitere Verarbeitung auf Tensidalkohole besonders günstige Komponenten und eine besonders vorteilhafte Zusammensetzung aufweisen, wenn man die Zusammensetzung des Nickel-Katalysators und die Reaktionsbedingungen so wählt, daß ein Dimerengemisch erhalten wird, welches weniger als 10 Gew.-% von Verbindungen enthält, die ein Strukturelement der Formel I (Vinylidengruppe) 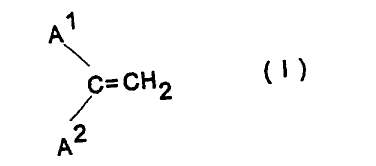 worin A¹ und A² aliphatische Kohlenwasserstoffreste sind,
aufweisen.

Die Dimerisierung kann homogenkatalysiert oder heterogenkatalysiert durchgeführt werden. Bevorzugt ist die heterogene Verfahrensweise, da hierbei einerseits die Katalysatorabtrennung vereinfacht und das Verfahren damit wirtschaftlicher ist, zum anderen werden keine umweltschädlichen Abwässer erzeugt, wie sie gewöhnlich bei der Abtrennung gelöster Katalysatoren, zum Beispiel durch Hydrolyse, anfallen. Ein weiterer Vorteil des heterogenen Verfahrens besteht darin, daß das Dimerisierungsprodukt keine Halogene, insbesondere Chlor oder Fluor, enthält. Homogen lösliche Katalysatoren enthalten im allgemeinen halogenidhaltige Liganden oder sie werden in Kombination mit halogenhaltigen Cokatalysatoren eingesetzt. Aus solchen Katalysatorsystemen kann Halogen in die Dimerisierungsprodukte eingebaut werden, was sowohl die Produktqualität als auch die Weiterverarbeitung, insbesondere die Hydroformylierung zu Tensidalkoholen erheblich beeinträchtigt.

Zur heterogene Katalyse werd zweckmäßigerweise Kombinationen von Nickeloxiden mit Aluminiumoxid auf Trägermaterialien aus Silizium- und Titanoxiden wie sie beispielsweise aus der DE-A-43 39 713 bekannt sind, eingesetzt. Der heterogene Katalysator kann im Festbett - dann vorzugsweise in grobkörniger Form als 1 bis 1,5 mm-Splitt - oder suspendiert (Partikelgröße 0.05 bis 0,5 mm) eingesetzt werden. Die Dimerisierung wird bei heterogener Durchführung zweckmäßigerweise bei Temperaturen von 80 bis 200°C, vorzugsweise von 100 bis 180°C, unter dem bei der Reaktionstemperatur herrschenden Druck, gegebenenfalls auch unter einem Schutzgasüberdruck, im geschlossenen System ausgeführt. Zur Erzielung optimaler Umsätze wird das Reaktionsgemisch zweckmäßigerweise mehrfach im Kreis geführt, wobei kontinuierlich ein bestimmter Anteil des zirkulierenden Produkts ausgeschleust und durch Ausgangsmaterial ersetzt wird.

Bei der erfindungsgemäßen Dimerisierung werden Mischungen einfach ungesättigter Kohlenwasserstoffe erhalten, deren Komponenten überwiegend die doppelte Kettenlänge haben wie die Ausgangs-Olefine.

Charakteristisch für die erfindungsgemäß hergestellten Olefingemische ist ihr hoher Anteil - in der Regel über 90%, insbesondere über 95% - von Komponenten mit Verzweigungen und der geringe Anteil - in der Regel unter 10%, insbesondere unter 5% - unverzweigter Olefine. Ein weiteres Charakteristikum ist, daß an den Verzweigungsstellen der Hauptkette überwiegend Methyl- oder Ethylgruppen gebunden sind.

Die nach dem Schritt a) des erfindungsgemäßen Verfahrens erhältlichen neuen Olefingemische mit den oben genannten Strukturmerkmalen sind ebenfalls ein Gegenstand der vorliegenden Erfindung. Sie stellen wertvolle Zwischenprodukte insbesondere für die gemäß Schritt b) des erfindungsgemäßen Verfahrens auszuführende und im Folgenden näher beschriebene Herstellung von verzweigten primären Alkoholen und Tensiden dar, können aber auch als Ausgangsmaterialien in anderen, von Olefinen ausgehenden technischen Prozessen eingesetzt werden, insbesondere dann, wenn die Endprodukte verbesserte toxikologische Eigenschaften haben sollen.

Sollen die erfindungsgemäßen Olefingemische zur Herstellung von Tensiden dienen, so werden sie zunächst gemäß Schritt b) des erfindungsgemäßen Verfahrens nach an sich bekannten Methoden zu Tensidalkoholen derivatisiert.

Hierzu bestehen verschiedene Wege, die entweder die direkte oder eine auf Umwegen erfolgende Anlagerung von Wasser (Hydratisierung) an die Doppelbindung oder eine Anlagerung von CO und Wasserstoff (Hydroformylierung) an die C=C-Doppelbindung umfassen.

Die Hydratisierung der aus dem Verfahrensschritt a) hervorgehenden Olefine erfolgt zweckmäßigerweise durch direkte Wasseranlagerung unter Protonenkatalyse. Natürlich ist auch ein Umweg, beispielsweise über die Addition von hochprozentiger Schwefelsäure zu einem Alkanolsulfat und anschließende Verseifung zum Alkanol möglich. Die zweckmäßigere direkte Wasseranlagerung wird in Gegenwart saurer, insbesondere heterogener, Katalysatoren und in der Regel bei möglichst hohem Olefin-Partialdruck und bei möglichst niedrigen Temperaturen durchgeführt. Als Katalysatoren bewähren sich insbesondere Phosphorsäure auf Trägern, wie z.B. SiO₂ oder Celite, oder auch saure Ionenaustauscher. Die Wahl der Bedingungen richtet sich nach der Reaktivität der umzusetzenden Olefine und kann routinemäßig durch Vorversuche ermittelt werden (Lit.: z.B. A. J. Kresge et al. J. Am. Chem.Soc. *93*, 4907 (1971); Houben-Weyl Bd. 5/4 (1960), Seiten 102-132 und 535-539). Die Hydratisierung führt in der Regel zu Mischungen von primären und sekundären Alkanolen, in denen die sekundären Alkanole überwiegen.

Für die Herstellung von Tensiden ist es günstiger, von primäre Alkanolen auszugehen. Es ist daher bevorzugt, die Derivatisierung - Schritt b) des erfindungsgemäßen Verfahrens - der aus Schritt a) erhaltenen Olefingemische durch Umsetzung derselben mit Kohlenmonoxid und Wasserstoff in Gegenwart von geeigneten, vorzugsweise kobalt- oder rhodiumhaltigen Katalysatoren zu verzweigten primären Alkoholen zu bewerkstelligen. (Hydroformylierung).

Eine gute Übersicht über das Verfahren der Hydroformylierung mit zahlreichen weiteren Literaturhinweisen findet sich beispielsweise in dem umfangreichen Aufsatz von Beller et al. in Journal of Molecular Catalysis, A104(1995) 17-85 oder in Ullmanns Encyclopedia of Industrial Chemistry, Bd.A5 (1986), Seite 217 ff., Seite 333, sowie die diesbezüglichen Literaturverweise.

Die dort gegebenen umfassenden Informationen ermöglichen es dem Fachmann, auch die in Schritt a) des erfindungsgemäßen Verfahrens erhaltenen Gemische verzweigter Olefine zu hydroformylieren. Bei dieser Reaktion wird CO und Wasserstoff an olefinische Doppelbindungen angelagert, wobei gemäß dem folgenden Reaktionsschema Mischungen aus Aldehyden und Alkanolen erhalten werden: 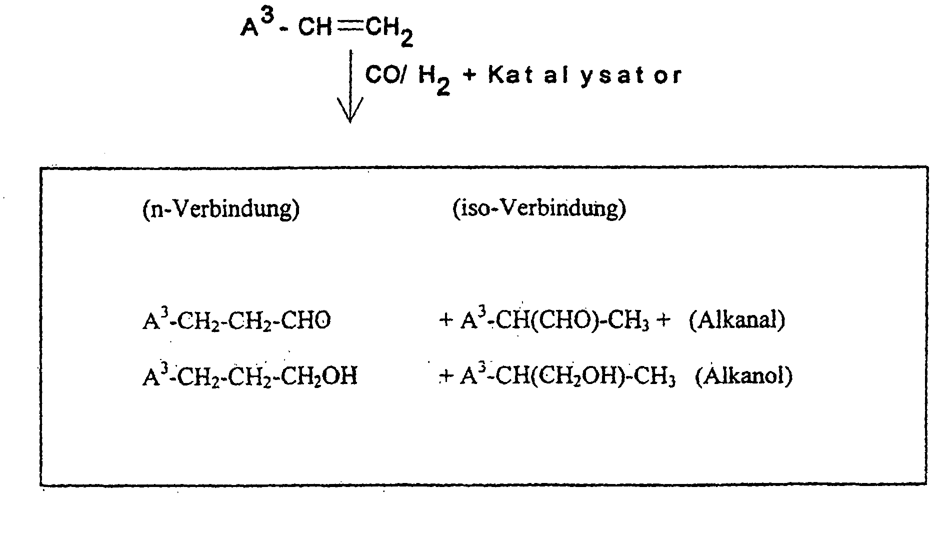 (A³ = Kohlenwasserstoffrest)

Das Molverhälnis von n- und iso-Verbindungen im Reaktionsgemisch liegt je nach den gewählten Verfahrensbedingungen der Hydroformylierung und dem eingesetzten Katalysator in der Regel im Bereich von 1:1 bis 20:1. Die Hydroformylierung wird normalerweise im Temperaturbereich von 90 bis 200° und bei einem CO/H₂-Druck von 2,5 bis 35 MPa (25 bis 350 bar) ausgeführt. Das Mischungsverhältnis von Kohlenmonoxid zu Wasserstoff richtet sich danach, ob vorzugsweise Alkanale oder Alkanole erzeugt werden sollen. Man arbeitet zweckmäßigerweise im Bereich CO:H₂ von 10:1 bis 1:10, vorzugsweise 3:1 bis 1:3, wobei man zur Herstellung von Alkanalen den Bereich der niedrigen Wasserstoffpartialdrucke, zur Herstellung von Alkanolen den Bereich der hohen Wasserstoffpartialdrucke, z.B. CO:H₂ = 1:2, wählt.

Als Katalysatoren eignen sich vor allem Metallverbindungen, der allgemeinen Formel HM(CO)₄ oder M₂(CO)₈, wobei M ein Metallatom, vorzugsweise ein Kobalt-, Rhodium- oder Rutheniumatom ist.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für einen Liganden, der ein Phosphin, Phosphit, Amin, Pyridin oder jede andere Donorverbindung, auch in polymerer Form, sein kann, und q, x, y und z für ganze Zahlen, abhängig von der Wertigkeit und Art des Metalls sowie der Bindigkeit des Liganden L, stehen, wobei q auch 0 sein kann.

Bei dem Metall M handelt es sich vorzugsweise um Kobalt, Ruthenium, Rhodium, Palladium, Platin, Osmium oder Iridium und insbesondere um Kobalt, Rhodium oder Ruthenium.

Geeignete Rhodiumverbindungen oder Komplexe sind z.B. Rhodium(II)- und Rhodium(III)-salze, wie Rhodium(III)-chlorid, Rhodium(III)-nitrat, Rhodium(III)-sulfat, Kalium-Rhodium-sulfat, Rhodium(II) bzw. Rhodium(III)-caboxylat, Rhodium(II)- und Rhodium(III)-acetat, Rhodium(III)-oxid, Salze der Rhodium(III)-säure, wie z.B. Trisammonium-hexachlororhodat-(III). Weiterhin eignen sich Rhodiumkomplexe wie Rhodiumbiscarbonyl-acetylacetonat, Acetylacetonatobisethylen-Rhodium-(I). Vorzugsweise werden Rhodiumbiscarbonyl-acetylacetonat oder Rhodiumacetat eingesetzt.

Geeignete Kobaltverbindungen sind zum Beispiel Kobalt(II)-chlorid, Kobalt(II)-sulfat, Kobalt(II)-carbonat, Kobalt(II)-nitrat, deren Amin- oder Hydratkomplexe, Kobaltcarboxylate wie Kobaltacetat, Kobaltethylhexanoat, Kobaltnaphthenat, sowie der Kobaltcaprolactamat-Komplex. Auch hier können die Carbonylkomplexe des Kobalts wie Dikobaltoctacarbonyl, Tetrakobaltdodecacarbonyl und Hexakobalthexadecacarbonyl eingesetzt werden.

Die genannten Verbindungen des Kobalts, Rhodiums und Rutheniums sind im Prinzip bekannt und in der Literatur hinreichend beschrieben, oder sie können vom Fachmann analog zu den bereits bekannten Verbindungen hergestellt werden.

Die Hydroformylierung kann unter Zusatz von inerten Lösungs- oder Verdünnungsmitteln oder ohne solchen Zusatz durchgeführt werden. Geeignete inerte Zusätze sind beispielsweise Aceton, Methyl-ethylketon, Cyclohexanon, Toluol, Xylol, Chlorbenzol, Methylenchlorid, Hexan, Petrolether, Acetonitril sowie die hochsiedenden Anteile aus der Hydroformylierung der Dimerisierungsprodukte.

Sofern das erhaltene Hydroformylierungsprodukt einen zu hohen Aldehydgehalt aufweist, kann dieser auf einfache Weise durch eine Hydrierung, zum Beispiel mit Wasserstoff in Gegenwart von Raney-Nickel oder unter Verwendung anderer für Hydrierungsreaktionen bekannter, insbesondere Kupfer, Zink, Kobalt, Nickel, Molybdän, Zirkon oder Titan enthaltender Katalysatoren , beseitigt werden. Dabei werden die Aldehydanteile weitgehend zu Alkanolen hydriert. Eine praktisch restlose Beseitigung von Aldehydanteilen im Reaktionsgemisch läßt sich gewünschtenfalls durch Nachhydrierung, beispielsweise unter besonders schonenden und ökonomischen Bedingungen mit einem Alkaliborhydrid, erreichen.

Die durch Hydroformylierung der aus Schritt a) des erfindungsgemäßen Verfahrens hervorgehenden neuen Olefingemische herstellbaren Gemische verzweigter primärer Alkanole sind ebenfalls ein Gegenstand der vorliegenden Erfindung.

Aus den erfindungsgemäßen Alkanolen können auf unterschiedliche Weise nichtionische oder anionische Tenside hergestellt werden.

Nichtionische Tenside werden erhalten durch Umsetzung der Alkanole mit Alkylenoxiden (Alkoxylierung) der Formel II 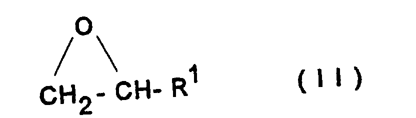 worin R¹ Wasserstoff oder ein geradkettiger oder verzweigter aliphatischer Rest der Formel CₙH₂ₙ₊₁ ist und n für eine Zahl von 1 bis 16, vorzugsweise von 1 bis 8 steht. Insbesondere bedeutet R¹ Wasserstoff, Methyl oder Ethyl.

Die erfindungsgemäßen Alkanole können mit einer einzigen Alkylenoxid-Species oder mit mehreren verschiedenen umgesetzt werden. Bei der Umsetzung der Alkanole mit den Alkylenoxiden entstehen Verbindungen, die wiederum eine OH-Gruppe tragen und daher erneut mit einem Alkylenoxidmolekül reagieren können. Es werden daher je nach dem Molverhältnis von Alkanol zu Alkylenoxid Reaktionsprodukte erhalten, die mehr oder weniger lange Polyetherketten aufweisen. Die Polyetherketten können 1 bis ca. 200 Alkylenoxid-Baugruppen enthalten. Bevorzugt sind Verbindungen, deren Polyetherketten 1 bis 10 Alkylenoxid-Baugruppen enthalten.

Die Ketten können aus gleichen Kettengliedern bestehen, oder sie können verschiedene Akylenoxid-Baugruppen aufweisen, die sich bezüglich ihres Restes R¹ voneinander unterscheiden. Diese unterschiedlichen Baugruppen können innnerhalb der Kette in statistischer Verteilung oder in Form von Blöcken vorliegen.

Das folgende Reaktionsschema soll die Alkoxylierung der erfindungsgemaßen Alkanole am Beispiel einer Umsetzung mit zwei verschiedenen Alkylenoxiden, die in unterschiedlichen Mol-Mengen x und y eingesetzt werden, veranschaulichen.

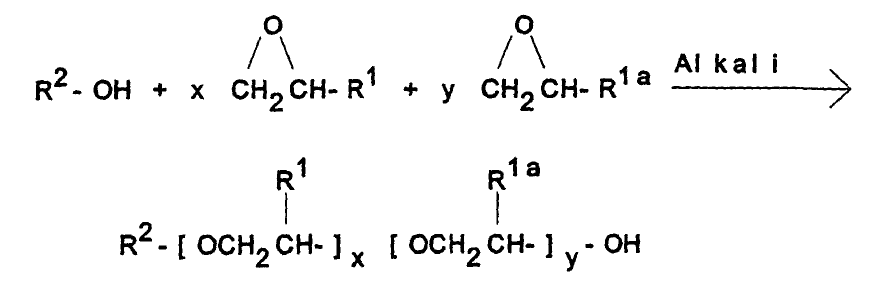

R¹ und R^{1a} sind verschiedene Reste im Rahmen der oben für R¹ gegebenen Definitionen und R²-OH ist ein erfindungsgemäßes verzweigtes Alkanol.

Vorzugsweise wird die Alköxylierung durch starke Basen katalysiert, die zweckmäßigerweise in Form eines Alkalihydroxids oder Erdalkalihydroxids, in der Regel in einer Menge von 0,1 bis 1 Gew.-% bezogen auf die Menge des Alkanols R²-OH, zugesetzt werden. (Vergl. G.Gee et al., J. Chem. Soc. (1961), S. 1345; B. Wojtech, Makromol. Chem. **66,** (1966), S.180)

Auch eine saure Katalyse der Additionsreaktion ist möglich. Neben Bronstedsäuren eignen sich auch Lewissäuren wie z.B. AlCl₃ oder BF₃. (Vergl. P.H-Plesch, The Chemistry of Cationic Polymerization, Pergamon Press, New York (1963)).

Die Additionsreaktion wird bei Temperaturen von etwa 120 bis etwa 220°C, vorzugsweise von 140 bis 160°C, im geschlossenen Gefäß ausgeführt. Das Alkylenoxid oder die Mischung verschiedener Alkylenoxide wird der Mischung aus erfindungsgemäßem Alkanolgemisch und Alkali unter dem bei der gewählten Reaktionstemperatur herrschenden Dampfdruck des Alkylenoxidgemisches zugeführt. Gewünschtenfalls kann das Alkylenoxid mit bis zu etwa 30 bis 60 % mit einem Inertgas verdünnt werden. Dadurch wird eine zusätzliche Sicherheit gegen explosionsartige Polyaddition des Alkylenoxids gegeben.

Wird ein Alkylenoxidgemisch eingesetzt, so werden Polyetherketten gebildet, in denen die verschiedenen Alkylenoxidbausteine praktisch statistisch verteilt sind. Variationen in der Verteilung der Bausteine längs der Polyetherkette ergeben sich aufgrund unterschiedlicher Reaktionsgeschwindigkeiten der Komponenten und können auch willkürlich durch kontinuierliche Zufuhr einer Alkylenoxidmischung programmgesteuerter Zusammensetzung erreicht werden. Werden die verschiedenen Alkylenoxide nacheinander zur Reaktion gebracht so erhält man Polyetherketten, mit blockartiger Verteilung der Alkylenoxid-Bausteine.

Die Länge der Polyetherketten schwankt innerhalb des Reaktionsprodukts statistisch um einen Mittelwert, der im wesentlichen dem sich aus der Zusatzmenge ergebenden stöchiometrischen Wert entspricht.

Die ausgehend von erfindungsgemäßen Olefingemischen und Alkanolgemischen herstellbaren Alkoxylate stellen ebenfalls einen Gegenstand der vorliegenden Erfindung dar. Sie zeigen eine senr gute Oberflächenaktivität und können daher als neutrale Tenside in vielen Anwendungsbereichen eingesetzt werden.

Ausgehend von den erfindungsgemäßen Alkanolgemischen können auch oberflächenaktive Glycoside und Polyglycoside (Oligoglycoside) hergestellt werden. Auch diese Substanzen zeigen sehr gute Tensideigenschaften. Sie werden erhalten durch ein- oder mehrfache Umsetzung (Glycosidierung bzw. Polyglycosidierung) der erfindungsgemäßen Alkanolgemische mit Mono-, Di- oder Poly-sacchariden unter Ausschluß von Wasser unter Säurekatalyse. Geeignete Säuren sind beispielsweise HCl oder H₂SO₄. In der Regel werden hierbei Oligoglycoside mit statistischer Kettenlängenverteilung erhalten, wobei der durchschnittliche Oligomerisierungsgrad bei 1 bis 3 Sacharid-Resten liegt.

Bei einer anderen Standardsynthese wird das Saccharid zunächst unter Säurekatalyse mit einem niedermolekularen Alkanol, z.B. Butanol, zum Butanolglycosid acetalisiert. Diese Reaktion kann auch mit wäßrigen Lösungen des Saccharids ausgeführt werden. Das. Niederalkanolglycosid, beispielsweise das Butanolglycosid, wird dann mit den erfindungsgemäßen Alkanolgemischen zu den gewünschten erfindungsgemäßen Glycosiden umgesetzt. Überschüssige lang- und kurzkettige Alkanole können nach Neutralisieren des sauren Katalysators aus dem Gleichgewichtsgemisch, z.B. durch Abdestillieren im Vakuum, entfernt werden.

Eine weitere Standardmethode verläuft über die O-Acetylverbindungen der Saccharide. Diese werden mit Halogenwasserstoff, der vorzugsweise in Eisessig gelöst ist, in die entsprechenden O-Acetyl-halosaccharide überführt, die in Gegenwart von Säure bindenden Agenzien mit den Alkanolen zu den acetylierten Glycosiden reagieren.

Bevorzugt für die Glycosidierung der erfindungsgemäßen Alkanolgemische sind Monosaccharide, und zwar sowohl die Hexosen wie Glucose, Fructose, Galactose, Mannose, als auch Pentosen wie Arabinose, Xylose oder Ribose. Besonders bevorzugt zur Glycosidierung der erfindungsgemäßen Alkanolgemische ist die Glucose. Selbstverständlich können auch Gemische der genannten Sacharide fiir die Glycosidierung eingesetzt werden. Es werden dann je nach den Reaktionsbedingungen Glycosiede mit statistisch verteilten Zuckerresten erhalten. Die. Glycosidierung kann auch mehrfach erfolgen, sodaß an die Hydroxylgruppen der Alkanole Polyglycosidketten angelagert werden. Bei einer Polyglycosidierung unter Einsatz verschiedener Saccharide können die Saccharid-Bausteine innerhalb der Kette statistisch verteilt sein oder Blöcke gleicher Baugruppen bilden.

Je nach der gewählten Reaktionstemperatur können Furanose- oder Pyranosestrukturen erhalten werden. Zur Verbesserung der Löslichkeitsverhältnisse kann die Reaktion auch in geeigneten Lösungs- oder Verdünnungsmitteln ausgeführt werden.

Standardverfahren und geeignete Reaktionsbedingungen sind in verschiedenen Publikationen beschrieben worden, beispielsweise in "Ullmanns Encyclopedia of Industrial Chemistry", 5.Auflage Bd. A25 (1994), Seiten 792-793 und in den dort angegebenen Literaturverweisen, von K.Igarashi, Adv. Carbohydr. Chem. Biochem. 34, (1977), S. 243-283, von Wulff und Röhle, Angew. Chem. 86, (1974), S.173-187, oder bei Krauch und Kunz, Reaktionen der organischen Chemie, S. 405-408, Hüthig, Heidelberg, (1976).

Die ausgehend von erfindungsgemäßen Olefingemischen und Alkanolgemischen herstellbaren Glycoside und Polyglycoside (Oligoglycoside) stellen ebenfalls einen Gegenstand der vorliegenden Erfindung dar.

Sowohl die erfindungsgemäßen Alkanolgemische als auch die daraus hergestellten Polyether können in anionische Tenside überführt werden, indem man sie in an sich bekannter Weise mit Schwefelsäure oder Schwefelsäurederivaten zu sauren Alkylsulfaten oder Alkylethersulfaten (sulfatiert) oder mit Phosphorsäure oder ihren Derivaten zu sauren Alkylphosphaten bzw. Alkyletherphosphaten verestert (phosphatiert).

Sulfatierungsreaktionen von Alkoholen sind bereits beschrieben worden z.B. in US-A-3 462 525, 3 420 875 oder 3 524 864. Details zur Durchführung dieser Reaktion finden sich auch in "Ullmanns Encyclopedia of Industrial Chemistry", 5.Auflage Bd. A25 (1994). Seiten 779-783 und in den dort angegebenen Literaturverweisen.

Wird Schwefelsäure selbst zur Veresterung eingesetzt, so verwendet man zweckmäßigerweise eine 75 bis 100 gew.-%ige, vorzugsweise 85 bis 98 gew.-%ige Säure (sog. "konzentrierte Schwefelsäure" oder "Monohydrat"). Die Veresterung kann in einem Lösungs- oder Verdünnungsmittel vorgenommen werden, wenn es für die Steuerung der Reaktion, z.B. der Wärmeentwicklung erwünscht ist. In der Regel wird der alkoholische Reaktant vorgelegt, und das Sulfatierungsmittel unter ständigem Durchmischen allmählich zugefügt. Wenn eine vollständige Veresterung der Alkoholkomponente gewünscht wird verwendet man das Sulfatierungsmittel und den Alkanol im Molverhältnis von 1:1 bis 1:1,5, vorzugsweise von 1:1 bis 1:1,2. Geringere Mengen von Sulfatierungsmittel können vorteilhaft sein, wenn Mischungen von erfindungsgemäßen Alkanol-alkoxylaten eingesetzt und Kombinationen von neutralen und anionischen Tensiden hergestellt werden sollen. Die Veresterung wird normalerweise bei Temperaturen von Zimmertemperatur bis 85°C, vorzugsweise im Bereich von 45 bis 75°C, ausgeführt.

Gegebenenfalls kann es zweckmäßig sein, die Veresterung in einem niedrig siedenden, mit Wasser nicht mischbaren Lösungs- und Verdünnungsmittel bei dessen Siedepunkt auszuführen, wobei das bei der Veresterung entstehende Wasser azeotrop abdestilliert wird.

Anstelle von Schwefelsäure der oben angegebenen Konzentration können zur Sulfatierung der erfindungsgemäßen Alkanolgemische auch beispielsweise Schwefeltrioxid, Schwefeltrioxid-Komplexe, Lösungen von Schwefeltrioxid in Schwefelsäure ("Oleum"), Chlorsulfonsäure, Sulfurylchlorid oder auch Amidosulfonsäure eingesetzt werden. Die Reaktionsbedingungen sind dann zweckentsprechend anzupassen.

Wird Schwefeltrioxid als Sulfatierungsmittel eingesetzt so kann die Reaktion auch vorteilhafterweise in einem Fallfilmreaktor im Gegenstrom, gewünschtenfalls auch kontinuierlich, ausgeführt werden.

Die Ansätze werden nach der Veresterung durch Alkalizusatz neutralisiert und, ggf. nach Entfernung überschüssigen Alkalisulfates und eventuell vorhandener Lösungsmittel, aufgearbeitet.

Die durch Sulfatierung erfindungsgemäßer Alkanole und Alkanolether und deren Gemische erhaltenen sauren Alkanolsulfate und Alkanolethersulfate und deren Salze sind ebenfalls ein Gegenstand der vorliegenden Erfindung.

In analoger Weise können erfindungsgemäße Alkanole und Alkanolether und deren Gemische mit Phosphatierungsagenzien auch zu sauren Phosphorsäureestern umgesetzt (phosphatiert) werden.

Als Phosphatierungsagenzien eignen sich vornehmlich Phosphorsäure, Polyphosphorsäure und Phosphorpentoxid, aber auch POCl₃, wenn anschließend eine Hydrolyse der verbleibenden Säurechloridfunktionen vorgenommen wird. Die Phosphatierung von Alkoholen ist beispielsweise in Synthesis **1985,** Seiten 449 bis 488 beschrieben worden.

Auch die durch Phosphatierung erfindungsgemäßer Alkanole und Alkanolether und deren Gemische erhaltenen sauren Alkanolphosphate und Alkanoletherphosphate sind ein Gegenstand der vorliegenden Erfindung.

Schließlich ist auch die Verwendung der ausgehend von den erfindungsgemäßen Olefingemischen herstellbaren Alkanolethergemische, Alkanolglycoside sowie die sauren Sulfate und Phosphate der Alkanolgemische und der Alkanolethergemische als Tenside ein Gegenstand der vorliegenden Erfindung.

Die folgenden Ausfiihrungsbeispiele veranschaulichen die Herstellung und Verwendung der erfindungsgemäßen Tenside.

### Beispiel 1.

### Dimerisierung von Hexenisomeren-Gemischen.

Ein isotherm beheizbarer Reaktor mit 16 mm Durchmesser wurde mit 100 ml eines Katalysators der folgenden Zusammensetzung befüllt:
50 Gew.-% NiO, 34 Gew.-% SiO₂, 13 Gew.-% TiO₂, 3 Gew.-% Al₂O₃ (gemäß DE-A-43 39 713), 24 Stdn. bei 160°C in N₂ konditioniert, eingesetzt als 1 bis 1,5 mm Splitt.

Mit zwei überwiegend Hexenisomeren enthaltenden Olefin-Gemischen unterschiedlicher Zusammensetzung wurden je 4 Versuche gemacht, wobei die Reaktionsbedingungen variiert wurden. Die Zusammensetzung der eingesetzten Olefin-Gemische ist in den Tabellen 1 und 2 angegeben; der Hexenisomeren-Anteil setzte sich jeweils zusammen aus 71 Gew.-% Methylpentenen, 22 Gew.-% n-Hexenen und 7 Gew.-% Dimethylbutenen.

Die Olefin-Mischungen wurden durch das festgelegte Katalysatorbett in einer auf das Reaktorvolumen bezogenen Rate (WHSV) von 0,25 kg/l*h geleitet und in einer Rate von 24 bis 28 g/h ausgeschleust. Variiert wurde bei den einzelnen Versuchen die Reaktionstemperatur, der Druck und die Betriebsdauer des Versuchs.

Die folgenden Tabellen 1 und 2 zeigen die Versuchsbedingungen der acht Versuche und die dabei erhaltenen Ergebnisse.

**Tabelle 1**

| Reaktionsbedingungen | | | | | |
|---|---|---|---|---|---|
| Temperatur [°C] | - | 100 | 120 | 140 | 160 |
| Druck [bar] | - | 20 | 20 | 20 | 25 |
| Versuchsdauer [Stdn.] | - | 12 | 19 | 36 | 60 |

| Zusammensetzung [Gew.-%] | | | | | |
|---|---|---|---|---|---|
| Komponenten | Feedstock | Reaktionsprodukte | | | |
| C₃ | 1,6 | - | - | - | - |
| C₆ | 73,1 | 39,9 | 24,0 | 28,9 | 32,1 |
| C₇ bis C₁₁ | 18,6 | 17,8 | 15,9 | 17,7 | 19,1 |
| C₁₂ | 5,1 | 31,7 | 44,6 | 42,6 | 39,2 |
| über C₁₃ | 1,6 | 10,6 | 15,5 | 10,8 | 9,6 |
| Schwefel [ppm] | 5 | - | - - | - | - |
| Chlor [ppm] | 6 | - | - | - | - |
| Umsatz* | - | 45,4 | 67,2 | 60,5 | 56,1 |
| C₁₂-Selek-tivität** | - | 80,2 | 80,4 | 84,8 | 83,2 |
| C₁₂-RZA*** | - | 67 | 99 | 94 | 85 |

| | | | | | |
|---|---|---|---|---|---|
| * = bezogen auf C₆, | | | | | |
| ** = C₁₂-anfangsgehalt-bereinigt, | | | | | |
| *** = bezogen auf den Durchsatz-Sollwert von 25 g/h | | | | | |

**Tabelle 2**

| Reaktionsbedingungen | | | | | |
|---|---|---|---|---|---|
| Temperatur [°C] | - | 100 | 120 | 140 | 160 |
| Druck [bar] | - | 20 | 20 | 20 | 25 |
| Versuchsdauer [Stdn.] | - | 12 | 19 | 36 | 60 |

| Zusammensetzung [Gew.-%] | | | | | |
|---|---|---|---|---|---|
| Komponenten | Feedstock | Reaktionsprodukte | | | |
| C₃ | - | - | - | - | - |
| C₆ | 98,3 | 64,2 | 33,5 | 26,9 | 31,7 |
| C₇ bis C₁₁ | 1,1 | 5,3 | 3,8 | 2,8 | 2,3 |
| C₁₂ | 0,6 | 27,0 | 54,4 | 61,3 | 58,5 |
| über C₁₃ | - | 3,5 | 8,3 | 9,0 | 7,5 |
| Schwefel [ppm] | <1 | - | - | - | - |
| Chlor [ppm] | <1 | - | - | - | - |
| Umsatz* | - | 34,7 | 65,9 | 72,6 | 67,8 |
| C₁₂-Selektivität** | - | 77,4 | 83,0 | 85,0 | 86,9 |
| C₁₂-RZA*** | - | 66 | 134 | 152 | 145 |

| | | | | | |
|---|---|---|---|---|---|
| * = bezogen auf C₆, | | | | | |
| ** = C₁₂-anfangsgehalt-bereinigt, | | | | | |
| *** = bezogen auf den Durchsatz-Sollwert von 25 g/h | | | | | |

Das ausgeschleuste Produkt wurde fraktioniert destilliert. Das Dodecengemisch hat einen Isoindex von 3,2 (Bestimmung NMR-Spektroskopisch nach Hydrierung).

### Beispiel 2, Hydroformylierung einer erfindungsgemäßen Dodecenmischung.

866 g eines gemäß Beispiel 1 hergestellten Dodecengemisches werden mit 3,26 g Co₂(CO)₈ bei 185°C und 280 bar CO/H₂ (Vol.-Verhältnis = 1:1,5) unter Zusatz von 87 g H₂O in einem 2,5 1 Hubrührautoklaven 5' Stunden hydroformyliert. Der Reaktionsaustrag wird mit 10 gew.-%iger Essigsäure unter Lufteinleitung bei 90°C oxidativ entkobaltet. Das Oxoprodukt wird unter Zusatz von 10 Gew.-% Wasser in einem 2,5 1 Rohrreaktor in Rieselfahrweise an einem Co/Mo-Festbettkatalysator bei 175°C und 280 bar Wasserstoffdruck unter Zusatz von 10 Gew.-% Wasser hydriert.

Das erhaltene Alkoholgemisch wird fraktioniert destilliert. Für das isolierte Tridecariolgemisch wird ¹H-NMR-spektrpskopisch ein Mittelwert von 4,4 Methylgruppen/Molekül bestimmt, entsprechend einem Verzweigungsgrad von 3,4.

### Beispiel 3, Herstellung eines Fettalkoholethoxilates mit 7 Mol/Mol Ethylenoxid.

400 g des gemäß Beispiel 2 hergestellten Alkanolgemisches werden mit 1,5 g NaOH in einen trockenen 2 1 - Autoklaven eingefüllt. Der Autoklaveninhalt wird auf 150°C erhitzt und 616 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator durch Schwefelsäurezusatz neutralisiert.

Das erhaltene Ethoxylat stellt ein neutrales Tensid dar. Es hat einen Trübungspunkt von 71°C, gemessen nach DIN 53917, 1 gew.-%-ig in 10 gew.-%iger wässriger Butyldiglykollösung. Die Oberflächenspannung einer Lösung von 1 g/l der Substanz in Wasser beträgt 26,1 mN/m, gemessen nach DIN 53914. Es zeigt eine für diese C-Kettenlänge sehr geringe Algentoxizität. Die EC₅₀-Werte liegen bei einem Ethoxylierungsgrad von 7 bei 22 mg/l/72h.

### Beispiel 4, Herstellung eines Fettalkoholethoxilates mit 3 Mol/Mol Ethylenoxid.

600 g des gemäß Beispiel 2 hergestellten Tridecanolgemisches werden mit 1,5 g NaOH in einen trockenen 2 1 - Autoklaven eingefüllt. Der Autoklaveninhalt wird auf 150°C erhitzt und 396 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator durch Schwefelsäurezusatz neutralisiert.

Das erhaltene Ethoxylat stellt ein neutrales Tensid dar. Es hat einen Trübungspunkt von 40,3°C, gemessen nach DIN 53917, 1 gew.-%-ig in 10 gew.-%iger wässriger Butyldiglykollösung. Die Oberflächenspannung einer Lösung von 1 g/l der Substanz in Wasser beträgt 25,7 mN/m, gemessen nach DIN 53914.

### Beispiel 5, Herstellung eines Alkylphosphats.

300 g des gemäß Beispiel 2 hergestellten Tridecanolgemisches werden in einem Rührgefäß unter Stickstoff auf 60°C erwärmt und langsam mit 125 g Polyphosphorsäure versetzt. Dabei soll die Temperatur 65°C nicht übersteigen. Gegen Ende der Zugabe wird der Ansatz auf 70°C erwärmt und eine Stunde bei dieser Temperatur weitergerührt. Das erhaltene Produkt stellt ein anionisches Tensid dar. Eine wässrige Lösung der Substanz in Wasser hat bei einer Konzentration von 1 g/l eine Oberflächenspannung von 28,9 mN/m, gemessen nach DIN 53914.

### Beispiel 6, Herstellung eines Alkyletherphosphats.

560 g des gemäß Beispiel 4 hergestellten Fettalkoholethoxylat-Gemisches werden in einem Rührgefäß unter Stickstoff auf 60°C erwärmt und langsam mit 92 g Polyphosphorsäure versetzt. Dabei soll die Temperatur 65°C nicht übersteigen. Gegen Ende der Zugabe wird der Ansatz auf 70°C erwärmt und eine Stunde bei dieser Temperatur weitergerührt.

Das erhaltene Produkt stellt ein anionisches Tensid dar. Eine wässrige Lösung der Substanz in Wasser hat bei einer Konzentration von 1 g/l eine Oberflächenspannung von 36,1 mN/m, gemessen nach DIN 53914.

### Beispiel 7, Herstellung eines Alkylsulfats.

190 g des gemäß Beispiel 2 hergestellten Tridecanolgemisches werden in einem Rührgefäß unter Stickstoff auf 60°C erwärmt und langsam mit 98 g konzentrierter Schwefelsäure versetzt. Dabei soll die Temperatur 65°C nicht übersteigen. Gegen Ende der Zugabe wird der Ansatz auf 70°C erwärmt und eine Stunde bei dieser Temperatur weitergerührt.

Das erhaltene Produkt stellt ein anionisches Tensid dar. Eine wässrige Lösung der Substanz in Wasser hat bei einer Konzentration von 1 g/l eine Oberflächenspannung von 29,8 mN/m, gemessen nach DIN 53914.

### Beispiel 8, Herstellung eines Alkylethersulfats.

480 g des gemäß Beispiel 4 hergestellten Fettalkoholethöxylat-Gemisches werden in einem Rührgefäß unter Stickstoff auf 60°C erwärmt und langsam mit 146 g konzentrierter Schwefelsäure versetzt. Dabei soll die Temperatur 65°C nicht übersteigen. Gegen Ende der Zugabe wird der Ansatz auf 70°C erwärmt und eine Stunde bei dieser Temperatur weitergerührt.

Das erhaltene Produkt stellt ein anionisches Tensid dar. Eine wässrige Lösung der Substanz in Wasser hat bei einer Konzentration von 1 g/l eine Oberflächenspannung von 35,2 mN/m, gemessen nach DIN 53914.

## Patentansprüche

1. Verfahren zur Herstellung von Tensidalkoholen und entsprechenden Tensidalkoholethern durch
a) Dimerisierung von Olefingemischen,
b) Derivatisierung zu primären Alkoholen, und
c) gegebenenfalls anschließende Alkoxylierung,
**dadurch gekennzeichnet, daß** man für die Dimerisierung einen Nickel enthaltenden Katalysator und ein Olefingemisch umfassend im wesentlichen C₆ bis C₁₂ - Olefine einsetzt, das mindestens 55 Gew.-% Hexenisomere enthält, wobei der Hexenisomerenanteil weniger als 30 Gew.-% lineare Isomere aufweist.

2. Verfahren gemäß Anspruch 1, **dadurch gekennzeichnet, daß** der Verfahrensschritt a), die Dimerisierung, heterogenkatalysiert durchgeführt wird.

3. Verfahren gemäß mindestens einem der Ansprüche 1 und 2, **dadurch gekennzeichnet, daß** das Olefingemisch mindestens 65 Gew.-% Hexenisomere enthält.

4. Verfahren gemäß mindestens einem der Ansprüche 1 bis 3, **dadurch gekennzeichnet, daß** man einen heterogenen Katalysator auf Basis einer Kombination von Nickeloxiden und Aluminiumoxid einsetzt, sodaß im Verfahrensschritt a) ein Dimerengemisch erhalten wird, welches weniger als 10 Gew.-% von Verbindungen enthält, die ein Strukturelement der Formel I (Vinylidengruppe) 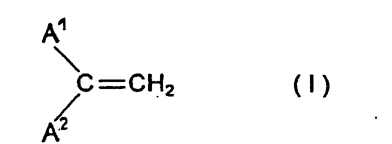 worin A¹ und A² aliphatische Kohlenwasserstoffreste sind, aufweisen.

5. Olefingemische, herstellbar nach dem Verfahrensschritt a) des Verfahrens des Anspruchs 1, **dadurch gekennzeichnet, daß** mindestens 90 Gew.-% der Komponenten des Dimerisierungsgemisches verzweigt sind, daß die verzweigten Komponenten des Dimerisierungsgemisches an den Verzweigungsstellen der Hauptkette überwiegend Methyl- oder Ethylgruppen tragen.

6. Olefingemische gemäß Anspruch 5, **dadurch gekennzeichnet, daß** sie weniger als 10 Gew.-% von Verbindungen enthalten, die ein Strukturelement der Formel I (Vinylidengruppe) 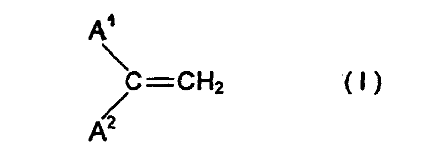 worin A¹ und A² aliphatische Kohlenwasserstoffreste sind, aufweisen.

7. Tensidalkohole und deren Alkoxylierungsprodukte, herstellbar nach den Verfahrensschritten a), b) und gegebenenfalls c) des Verfahrens des Anspruchs 1, **dadurch gekennzeichnet, daß** im Verfahrensschritt b) und gegebenenfalls c) ein Olefingemisch gemäß Anspruch 5 eingesetzt wird.

8. Verwendung der Tensidalkohol-Alkoxylierungsprodukte des Anspruchs 7 als nichtionische Tenside.

9. Verwendung der Tensidalkohole des Anspruchs 7 zur Herstellung von Tensiden.

10. Verwendung der Tensidalkohole oder deren Alkoxylierungsprodukte des Anspruchs 7 zur Herstellung von Alkanolglycosid- und -polyglycosid-Gemischen durch ein- oder mehrfache Umsetzung (Glycosidierung, Polyglycosidierung) derselben mit Mono-, Di- oder Poly-sacchariden unter Ausschluß von Wasser unter Säurekatalyse oder mit O-Acetylsaccharid-haliden, oder
zur Herstellung oberflächenaktiver Sulfate durch Veresterung derselben mit Schwefelsäure oder Schwefelsäurederivaten zu sauren Alkylsulfaten oder Alkylethersulfaten, oder
zur Herstellung oberflächenaktiver Phosphate durch Veresterung derselben mit Phosphorsäure oder ihren Derivaten zu sauren Alkylphosphaten bzw. Alkyletherphosphaten

## Claims

1. A process for the preparation of surfactant alcohols and corresponding surfactant alcohol ethers by
a) dimerization of olefin mixtures,
b) derivatization to give primary alcohols, and
c) optional subsequent alkoxylation,
which comprises using for the dimerization a catalyst containing nickel, and an olefin mixture comprising essentially C₆- to C₁₂-olefins, which comprises at least 55% by weight of hexene isomers, where the hexene isomer fraction has less than 30% by weight of linear isomers.

2. A process as claimed in claim 1, wherein process step a), the dimerization, is carried out with heterogeneous catalysis.

3. A process as claimed in at least one of claims 1 and 2, wherein the olefin mixture comprises at least 65% by weight of hexene isomers.

4. A process as claimed in at least one of claims 1 to 3, wherein a heterogeneous catalyst based on a combination of nickel oxides and aluminium oxide is employed so that, in process step a), a dimer mixture is obtained which comprises less than 10% by weight of compounds which have a structural element of the formula I (vinylidene group) 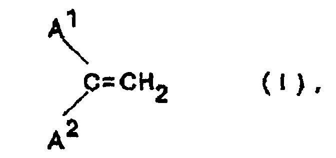 in which A¹ and A² are aliphatic hydrocarbon radicals.

5. An olefin mixture as claimed in at least one of claims 5 and 6, wherein the branched components of the dimerization mixture carry predominantly methyl or ethyl groups at the branching sites of the main chain.

6. An olefin mixture as claimed in claim 5, which comprises less than 10% by weight of compounds which have a structural element of the formula I (vinylidene group) 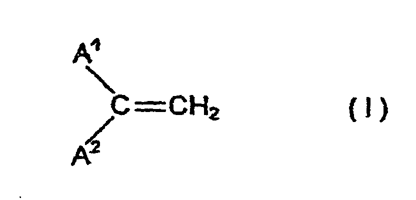 in which A¹ and A² are aliphatic hydrocarbon radicals.

7. A surfactant alcohol or alkoxylation product thereof, preparable by process steps a), b) and, if appropriate, c) of the process of claim 1, wherein, in process step b) and, if appropriate, c), an olefin mixture as claimed in claim 5 is employed.

8. The use of the surfactant alcohol alkoxylation products of claim 7 as nonionic surfactants.

9. The use of the surfactant alcohols of claim 7 for the preparation of surfactants.

10. The use of the surfactant alcohols and alkoxylation products thereof as claimed in claim 7 for the preparation of alkanol glycoside and polyglycoside mixtures by single or multiple reaction (glycosidation, polyglycosidation) with mono-, di- or polysaccharides with the exclusion of water and with acid catalysis or with O-acetylsaccharide halides, or for the preparation of surface-active sulfates by esterification thereof with sulfuric acid or sulfuric acid derivatives to give acidic alkyl sulfates or alkyl ether sulfates, or for the preparation of surface-active phosphates by esterification thereof with phosphoric acid or its derivatives to give acidic alkyl phosphates or alkyl ether phosphates.

## Revendications

1. Procédé de préparation d'alcools tensioactifs et d'éthers d'alcools tensioactifs correspondants par
a) dimérisation de mélanges d'oléfines,
b) dérivatisation en alcools primaires, et
c) éventuellement alcoxylation subséquente,
**caractérisé en ce que** l'on met en oeuvre pour la dimérisation un catalyseur contenant du nickel et un mélange d'oléfines comprenant essentiellement des oléfines en C₆ à C₁₂, qui contient au moins 55% en poids d'isomères de l'hexène, la fraction d'isomères d'hexène présentant moins de 30% en poids d'isomères linéaires.

2. Procédé selon la revendication 1, **caractérisé en ce que** l'étape de procédé a), la dimérisation, est entreprise avec catalyse hétérogène.

3. Procédé selon l'une au moins des revendications 1 et 2, **caractérisé en ce que** le mélange d'oléfines contient au moins 65% en poids d'isomères de l'hexène.

4. Procédé selon l'une au moins des revendications 1 à 3, **caractérisé en ce que** l'on met en oeuvre un catalyseur hétérogène à base d'une combinaison d'oxydes de nickel et d'oxyde d'aluminium, de sorte que dans l'étape de procédé a), on obtient un mélange de dimères qui contient moins de 10% en poids de composés présentant un élément structurel de la formule I (groupe vinylidène) 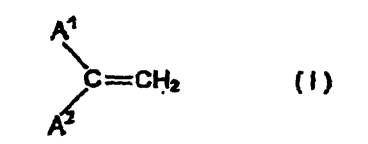 dans laquelle A¹ et A² sont des restes hydrocarbonés aliphatiques.

5. Mélanges d'oléfines, qui peuvent être préparés selon l'étape de procédé a) du procédé de la revendication 1, **caractérisés en ce qu'**au moins 90% en poids des composants de la dimérisation sont ramifiés, que les composants ramifiés du mélange de dimérisation portent principalement aux sites de ramification de la chaîne principale des groupes méthyle ou éthyle.

6. Mélanges d'oléfines selon la revendication 5, **caractérisés en ce qu'**ils contiennent moins de 10% en poids de composés présentant un élément structurel de la formule I (groupe vinylidéne) 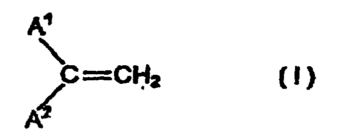 dans laquelle A¹ et A² sont des restes hydrocarbonés aliphatiques.

7. Alcools tensioactifs et leurs produits d'alcoxylation, qui peuvent être préparés selon les étapes de processus a), b) et éventuellement c) de la revendication 1, **caractérisés en ce que**, dans l'étape de processus b) et éventuellement c), on met en oeuvre un mélange d'oléfines selon la revendication 5.

8. Utilisation des produits d'alcoxylation d'alcools tensioactifs de la revendication 7 comme tensioactifs non ioniques.

9. Utilisation des alcools tensioactifs de la revendication 7 pour la préparation de tensioactifs.

10. Utilisation des alcools tensioactifs ou de leurs produits d'alcoxylation selon la revendication 7 pour la préparation de mélanges de glycosides et de polyglycosides d'alcanols par réaction simple ou multiple (glycosidation, polyglycosidation) de ces composés avec des mono-, di- ou polysaccharides en l'absenoe d'eau et avec catalyse acide ou avec des halogénures d'O-acétylsaccharides,
ou pour la préparation de sulfates tensioactifs par estérification de ces composés avec de l'acide sulfurique ou des dérivés d'acide sulfurique pour former des sulfates d'alkyle ou des éther sulfates d'alkyle acides,
ou pour la préparation de phosphate tensioactifs par estérification de ces composés avec de l'acide phosphorique ou ses dérivés pour former des phosphates d'alkyle ou des éther phosphates d'alkyle acides.
